(19) Europäisches Patentamt
European Patent Office
Office européen des brevets

(11) Veröffentlichungsnummer: **0 012 967**
**B1**

# (12) EUROPÄISCHE PATENTSCHRIFT

(45) Veröffentlichungstag der Patentschrift: **28.12.83**

(51) Int. Cl.³: **A 61 K 49/02,**
C 07 C 103/50
//C07C103/365

(21) Anmeldenummer: **79105212.9**

(22) Anmeldetag: **17.12.79**

**(54) (2,4,5-Trimethylacetanilido)-imino-diacetat, Technetium-99m-markiertes (2,4,5-Trimethylacetanilido)-iminodiacetat zur Leberfunktionsdiagnostik und Verfahren zu deren Herstellung.**

(30) Priorität: **21.12.78 DE 2855334**

(43) Veröffentlichungstag der Anmeldung:
**09.07.80 Patentblatt 80/14**

(45) Bekanntmachung des Hinweises auf die Patenterteilung:
**28.12.83 Patentblatt 83/52**

(84) Benannte Vertragsstaaten:
**AT BE CH DE FR GB IT LU NL SE**

(56) Entgegenhaltungen:
**DE - A - 2 612 698**
**DE - A - 2 723 605**
**DE - A - 2 913 173**

**NUC COMPACT, COMPACT NEWS IN NUCLEAR MEDICINE, Band 10, September 1979, Seiten 147,148,150 J. MAHLSTEDT et al.: "Neue Iminodiazetessigsäure (IDA)-Derivate zur hepatobiliären Funktionszintigraphie (HBFS)"**

(73) Patentinhaber: **HOECHST AKTIENGESELLSCHAFT**
**Postfach 80 03 20**
**D-6230 Frankfurt am Main 80 (DE)**

(72) Erfinder: **Molter, Michael, Dr.**
**Unter den Akazien 7**
**D-6000 Frankfurt am Main 79 (DE)**
Erfinder: **Kloss, Gerhard, Dr.**
**Liederbachstrasse 14**
**D-6233 Kelkheim (Taunus) (DE)**
Erfinder: **Schickel, Eberhard**
**Am Weidenbruch 24**
**D-6251 Selters/Taunus (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

EP 0 012 967 B1

(2,4,5-Trimethylacetanilido)-imino-diacetat, Technetium-99m-markiertes (2,4,5-Trimethylacetanilido)-iminodiacetat zur Leberfuntionsdiagnostik und Verfahren zu deren Herstellung

Die vorligende Erfindung bezieht sich auf ein neues Präparat zur szintigraphischen Darstellung des hepatobiliären Systems (Leber, Gallenblase, intra- und extrahepatische Gallenwege). Das Präparat ist vor allem für dynamische Untersuchungen geeignet.

Für die nuklearmedizinische Diagnostik hat sich in den letzten Jahren Technetium-99m wegen seiner günstigen physikalischen Parameter (keine Korpuskularstrahlung, $\gamma$-Energie von 140 keV, Halbwertszeit von 6 h) und der damit verbundenen geringen Strahlenbelastung für Patient und Personal, sowie wegen der einfachen Gewinnung mit Hilfe von Nuklidgeneratoren weitgehend durchgesetzt.

Das aus solchen Generatoren gewonnene Technetium-99m liegt zunächst als Pertechnetat vor und ist in dieser Form, z.B. für die Schilddrüsen- und Hirnszintigraphie geeignet. Um auch andere Organe der Diagnostik mit Tc-99m zugänglich zu machen, wurden organspezifische Transportsubstanzen entwickelt, die gut mit Tc-99m markiert werden können und die so eine gute szintigraphische Darstellung der verschiedensten Organe zulassen. So kann man z.B. mit markierten Kolloiden die RES-haltigen Organe wie Leber und Milz darstellen; für die Knochenszintigraphie eigen sich bestimmte markierte Phosphorverbindungen.

Für die Markierung der Transportsubstanzen mit Tc-99m wird im allgemeinen das sehr reaktionsräge Pertechnetat ($TcO_4^-$) zunächst in eine niedrigere oxidationsstufe reduziert. In dieser Form ist Technetium dann reaktionsfreudig und bildet mit der entsprechenden Transportsubstanz einen relativ stabilen Komplex.

Die Reduktion des $TcO_4^-$ kann durch chemische Reduktionsmittel oder elektrolytisch durchgeführt werden; die Reduktion mit Zinn(II)-salzen ist heute am weitesten verbreitet.

Die Reduktion mit Zinn(II)-Verbindungen hat den Vorteil, daß man das Reduktionsmittel und die organspezifische Transportsubstanz — im allgemeinen in gefriergetrochneter Form — in einer Injektionsflasche gemeinsam aufbewahren kann, so daß für die Herstellung des anwendungsfertigen Präparats in der Klinik lediglich das Generatoreluat zugegeben werden muß, welches das $^{99m}$Tc-Pertechnetat enthält.

Durch die zur Verfügung stehenden Detektoren war die nuklearmedizinische Diagnostik lange Zeit weitgehend auf die Lokalisation bestimmter Organe und die Feststellung von Veränderungen an ihnen beschränkt. Durch die Weiterentwicklung der $\gamma$-Kamera und ihre Kopplung mit elektronischen Datenverarbeitungsanlagen ist es möglich geworden, Szintigramme in Sekundenabständen aufzunehmen und zu speichern. Mit Hilfe solcher Szintigramm-Sequenzen kann man neben der bildlichen Darstellung eines bestimmten organs, z.B. der Leber, auch Aussagen über dessen Funktion machen. Man spricht dann von "Funktionsszintigraphie".

Für Leberfunktionsuntersuchungen verwendete man zunächst mit J-131 markierte lebergängige Substanzen wie z.B. $^{131}$J-Bengalrosa oder $^{131}$J-Bromsulfan. Wegen der relativ hohen Strahlenbelastung und wegen der bei diesen Verbindungen vergleichsweise langsamen Leberpassage, die aus untersuchungstechnischen Gründen unvorteilhaft ist, wurde nach Wegen gesucht, für diese Untersuchungen ein mit Technetium-99m markierbares Diagnostikum mit schnellerer Leberpassage zu finden.

In der Literatur wurden hauptsächlich drei verschiedene Substanzen bzw. Substanzklassen für solche Untersuchungen vorgeschlagen:

1. $^{99m}$Tc-Penicillamin (z.B. G.T. Krishnamurathy, M. Tubis, J.S. Endow et al. J. Nucl. Med. *13*, 447 (1972))
2. $^{99m}$Tc-Pyridoxalaminosäuren, insbesondere $^{99m}$Tc-Pyridoxalglutamat (z.B. R. J. Baker, J. C. Bellen, P. M. Ronai: J. Nucl. Med. *16*, 720 (1975))
3. $^{99m}$Tc-Acetanilido-iminodiacetat (z.B. E. Harvey, M. Loberg, M. Cooper: J. Nucl. Med. *16*, 533 (1975))

Bei den $^{99m}$Tc-Acetanilido-iminodiacetaten fanden zunächst die beiden Derivate (2,6-Dimethylacetanilido)-iminodiacetat (HIDA) und (2,6-Diäthylacetanilido)-iminodiacetat (EHIDA) eine breite Anwendung.

In der DE—A—27 23 605 sind die Verbindungen der Formel

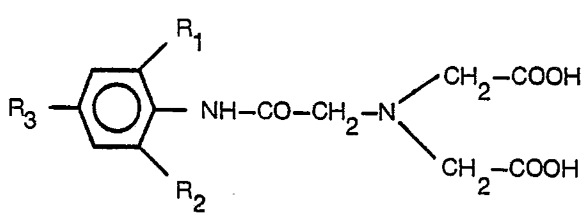

beschrieben, "in welcher mindestens zwei der Symbole $R_1$, $R_2$ und $R_3$ niedere Alkylgruppen mit 1 bis 4 Kohlenstoffatomen, das dritte eine Wasserstoffatomen oder eine niedrige Alkylgruppe mit 1 bis 4 Kohlenstoffatomen bedeuten und die drei Symbole insgesamt mindestens drei Kohlenstoffatome darstellen". Dabei ist speziell auf die Verbindungen (2,6-Diäthylacetanilido)-iminodiacetat, (2,6-Diisopropylacetanilido)-iminodiacetat und (2,4,6-Trimethylacetanilido)-iminodiacetat abgehoben.

Alle bisher beschriebenen Derivate des Acetanilid-iminodiacetats werden zu einem bestimmten Anteil über die Niere ausgeschieden, so daß bei der Anwendung auch die Niere deutlich dargestellt wird.

Aufgabe der vorliegenden Erfindung ist die Bereitstellung und Herstellung eines Leberfunktionsdiagnostikums mit möglichst geringer Nierenausscheidung und einer besonders schnellen Leberpassage, um die für die Untersuchung benötigte Zeit so kurz wie möglich zu halten.

Die Erfindung betrifft zunächst (2,4,5-Trimethylacetanilido)-iminodiacetat und ein Verfahren zu seiner Herstellung, das dadurch gekennzeichnet ist, daß man Chloressigsäure-(2,4,5-trimethylanilid) im alkalischen Medium mit Iminodiessigsäure umsetzt.

Die Erfindung ist auch ein Diagnostikum zur Darstellung des hepato-biliären Systems, das ein mit Technetium-99m markiertes Acetanilido-iminodiacetat (IDA) in einem geeigneten Lösungsmittel enthält, und das dadurch gekennzeichnet ist, daß das IDA (2,4,5-Trimethylacetanilido)-iminodiacetat ist. Als Lösungsmittel wird physiologische Kochsalzlösung bevorzugt.

Das Diagnostikum wird vorzugsweise in einer Dosierung von 0,04 bis 0,4 mg/kg Körpergewicht i.V. appliziert. Eine Lösung in physiologischer Kochsalzlösung wird dabei bevorzugt. Eine Markierungseinheit enthält zweckmäßig 30 mg. Das neue Diagnostikum zeichnet sich gegenüber den bisher bekannten Präparaten durch eine schnellere Leberpassage und eine geringere Anreicherung von Aktivität in der Niere aus. Dies Vorteile gehen aus den im filgenden beschriebenen Tierexperimenten hervor:

1. Einem Kanischen werden 0,5 mCi des jeweiligen markierten Diagnostikums in die Ohrvene injiziert und die Verteilung der Aktivität mit Hilfe einer $\gamma$-Kamera, die an eine Datenverarbeitungsanlage angeschlossen ist, in ihrem zeitlichen Verlauf verfolgt. Es wird dann der Verlauf der Aktivität über bestimmten Organen verfolgt. Dabei ermittelt man unter anderem folgende Parameter:

1. $Leber_{max.}$ = Zeit der maximalen Leberaktivität nach der Injektion
2. $Leber_{max.}$ = Halbwertszeit der Aktivität in der Leber (außerhalb der Gallenblase)

Diese Werte für einige Derivate des Acetanilido-iminodiacetats sind in der folgenden Tabelle angegeben:

| Substitution am Aromaten | | $Leber_{max}$ | $Leber_{max/2}$ |
|---|---|---|---|
| 2,6-Dimethyl- | (HIDA) | 5,7 min. | 8,2 min. |
| 2,6-Diäthyl- | (EHIDA) | 6,8 min. | 15,7 min. |
| 2,6-Diisopropyl- | | 4,6 min. | 15,3 min. |
| 2,4,6-Trimethyl | | 4,2 min. | 10,4 min. |
| 2,4,5-Trimethyl- | (TMIDA) | 2,6 min. | 7,7 min. |

2. Gruppen von jeweils 12 Ratten werden je 30 $\mu$Ci des jeweiligen markierten Diagnostikums in die Oberschenkelvene injiziert. Dann werden je 3 Tiere nach 5, 10, 20 und 30 Minuten getötet und die Organverteilung der Radioaktivität bestimmt. In der folgenden Tabelle sind die Ergebnisse zusammensgefäßt:

| Substitution am Aromaten | Zeit | Leber | Darm | Nieren |
|---|---|---|---|---|
| 2,6-Dimethyl- (HIDA) | 5' | 21,7% | 43,2% | 7,4% |
| | 10' | 9,6% | 65,9% | 7,4% |
| | 20' | 2,2% | 80,8% | 5,8% |
| | 30' | 1,0% | 79.7% | 4,9% |
| 2,6-Diäthyl- (EHIDA) | 5' | 6,9% | 71,2% | 4,3% |
| | 10' | 3,4% | 79,9% | 4,2% |
| | 20' | 1,6% | 81,7% | 3,6% |
| | 30' | 1,5% | 81,8% | 3,4% |
| 2,6-Diisopropyl- | 5' | 10,0% | 60,9% | 4,4% |
| | 10' | 7,0% | 65,3% | 4,4% |
| | 20' | 4,4% | 71,9% | 4,4% |
| | 30' | 4,4% | 72,2% | 3,8% |
| 2,4,6-Trimethyl- | 5' | 23,5% | 57,0% | 3,6% |
| | 10' | 12,1% | 66,0% | 3,8% |
| | 20' | 2,9% | 79,2% | 3,8% |
| | 30' | 1,6% | 81,0% | 3,3% |
| 2,4,5-Trimethyl- (TMIDA) | 5' | 19,1% | 56,8% | 3,3% |
| | 10' | 9,6% | 68,8% | 3,1% |
| | 20' | 2,4% | 81,1% | 2,5% |
| | 30' | 1,2% | 82,0% | 2,1% |

Die Dosis tolerata maxima des Diagnostikums beträgt bei Mäusen und Ratten 189 mg/kg Körpergewicht, die $LD_{50}$ beträgt an der Maus 246 mg und an der Ratte 255 mg pro Kilogramm Versuchstier.

Die Erfindung betrifft ferner ein Verfahren zur Herstellung des Leberfuntionsdiagnostikums, wobei man ein Acetanilidoiminodiacetat (IDA) in wäßriger Lösung mit einer Zinn(II)-Salz im Molverhältnis zwischen 10:1 und 200:1 mischt, die Lösung auf einen pH-Wert zwischen 4 und 9, vorzugsweise zwischen 5,0 und 6,5 einstellt und anschließend je nach Verwendungszweck 0,1—100 mCi Technetium-99m-Pertechnetat in einem geeigneten Lösungsmittel, vorzugsweise in physiologischer Kochsalzlösung, zugibt, das dadurch gekennzeichnet ist, daß man als IDA (2,4,5-Trimethylacetanilido)-iminodiacetat (TMIDA) verwendet. Die Konzentration von TMIDA liegt zweckmäßig zwischen 0,1 und 200 mg/ml, vorzugsweise zwischen 10 und 50 mg/ml.

Für die Herstellung des Diagnostikums ist es zweckmäßig, wenn die (2,4,5-Trimethylacetanilido)-iminodiessigsäure unter Zugabe von wäßriger Natronlauge in Wasser gelöst wird. Es wird dabei soviel NaOH zugegeben, wie zum vollständigen Lösen der Verbindung erforderlich ist. Der pH-Wert liegt nach dem Lösen zwischen 7 und 10; er wird dann mit HCl auf ca. 5 eingestellt. Danach wird die entsprechende Menge Sn(II)-Verbindung (in 0,1 n HCl gelöst) zugegeben und 30 Minuten unter Schutzgasatmosphäre gerührt und der pH auf 6 eingestellt.

Diese Lösung kann innerhalb von 24 Stunden durch Zugabe von Technetium-99m-Pertechnetat zum gebrauchsfertigen Diagnostikum eingesetzt werden. Sie kann aber auch zur Stabilisierung eingefroren oder gefriergetrocknet werden. Das gefriergetrocknete Präparat ist unter Stickstoff-Schutzgas mindestens 8 Monate stabil.

## Beispiel 1

Herstellung von 2,4,5-Trimethylacetanilido)-iminodiessigsäure

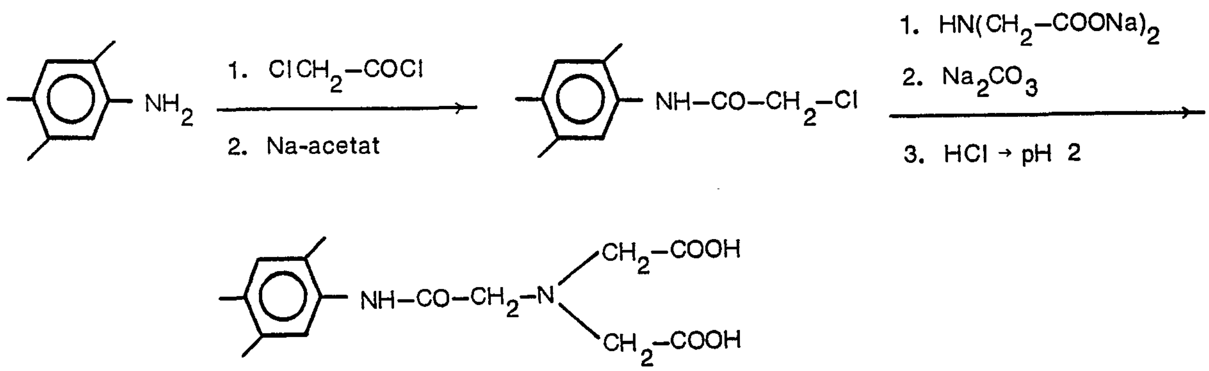


a) 13,52 g (0,1 mol) 2,4,5-Trimethylanilin werden in 80 ml Eisessig gelöst. Die Lösung wird auf 10°C gekühlt, mit 12,4 g (0,11 mol) Chloressigsäurechlorid versetzt und kräftig durchmischt. Dann werden unter starkem Rühren 100 ml halb gesättigte wäßrige Natriumacetat-Lösung und 100 ml Wasser zugegeben und 30 Minuten gerührt. Das ausgefallene Chloressigsäure-(2,4,5-Trimethylanilid) wird abgesaugt und aus Methanol umkristallisiert.
Schmelzpunkt: 163—165°C

| Elementaranalyse: | C | H | N | Cl |
|---|---|---|---|---|
| ber. | 62,41% | 6,67% | 6,62% | 16,75% |
| gef. | 62,5 % | 6,6 % | 6,7 % | 16,8 % |

NMR (in DMSO-$d_6$, interner Standard: TMS):

—$CH_2$—: $\delta = 4{,}23$ ppm(s)
—NH—: $\delta = 9{,}48$ ppm(s)
Arom.:3-H: $\delta = 6{,}94$ ppm(s)
6-H: $\delta = 7{,}09$ ppm(s)
2-$CH_3$: $\delta = 2{,}10$ ppm(s)
4-$CH_3$: $\delta = 2{,}15$ ppm(s)
5-$CH_3$: $\delta = 2{,}15$ ppm(s)

b) 4,0 g (22 mmol) Iminodiessigsäure-Dinatriumsalz, 5,7 g (20 mmol) $Na_2CO_3 \cdot 10H_2O$ und 4,25 g (20 mmol) Chloressigsäure-(2,4,5-Trimethylanilid) werden in 100 ml Wasser und 100 ml Äthanol aufgenommen und 24 Stunden am Rückfluß gekocht. Nach dem Abkühlen wird das Lösungsmittel abgezogen und der Rückstand in ca. 150 ml Wasser gelöst. Diese wäßrige Lösung wird 4 x mit ca. 50 ml Äther gewaschen und anschließend mit HCl langsam auf pH 2 gebracht. Das angefallene Produkt wird abgesaugt, wieder in Wasser gelöst, wobei soviel NaOH zugegeben wird, wie zum vollständigen Lösen der Substanz erforderlich ist. Nach Filtrieren wird (2,4,5-Trimethylacetanilido)-iminodiessigsäure durch Zugabe von HCl bis pH 2 wieder ausgefällt, abgesaugt und getrocknet. Schmelzpunkt: 196—198°C

| Elementaranalyse: | C | H | N |
|---|---|---|---|
| ber. | 58,43% | 6,54% | 9,09% |
| gef. | 58,3 % | 6,6 % | 9,0 % |

NMR (in DMSO-$d_6$, int. Standard: TMS):

| | |
|---|---|
| >N—$CH_2$—COOH: | $\delta = 3{,}55$ ppm(s) |
| —CO—$CH_2$—N<: | $\delta = 3{,}42$ ppm(s) |
| —NH—: | $\delta = 9{,}49$ ppm(s) |
| —COOH: | $\delta = 12{,}5$ ppm(s) |
| Arom.: 3-H: | $\delta = 6{,}95$ ppm(s) |
| 6-H: | $\delta = 7{,}56$ ppm(s) |
| 2-,4-,5-$CH_3$: | $\delta = 2{,}15$ ppm(s) |

### Beispiel 2

1,8 g (2,4,5-Trimethylacetanilido)-iminodiessigsäure werden in 20 ml 0,5 N Natronlauge gelöst und mit 36 mg $SnCl_2 \cdot 2H_2O$ (gelöst in 6 ml 0,1 N Salzsäure) versetzt. Der Ansatz wird 5 min. gerührt, mit Salzsäure auf pH 6 eingestellt, auf 60 ml aufgefüllt und in 1 ml-Portionen vereinzelt. Einer solchen Portion werden dann 10 mCi Tc-99m-Pertechnetat in 4 ml physilog. Kochsalzlösung zugesetzt. Nach 30 Minuten werden 0,5 ml des so hergestellten Diagnostikums einem Kaninchen intravenös appliziert (einge Daten dieses Versuchs können der entsprechenden Tabelle entnommen werden). Alle Arbeiten wurden unter $N_2$-Schutzgas durchgeführt.

### Beispiel 3

7 g (2,4,5-Trimethylacetanilido)-iminodiessigsäure werden in 100 ml 1 N Natronlauge gelöst und mit 16,5 mg $SnF_2$ (gelöst in 5 ml 0,1 N Salzsäure) versetzt. Nach 5 Minuten Rühren wird der pH-Wert mit Salzsäure auf 6 eingestellt, auf 233 ml aufgefüllt, in 1 ml-Portionen in Rollrandflaschen abgefüllt und mit flüssigem Stickstoff eingefroren. Anschließend wird das Präparat gefriergetrocknet, die Flaschen werden mit Stickstoff gefüllt und verschlossen.

Nach einer Lagerzeit von 60 Tagen werden in die verschlossenen Flaschen 0,3 mCi Tc-99m-Pertechnetat in 100 ml physiologischer Kochsalzlösung injiziert. Nach 20 Minuten Reaktionszeit werden je 0,5 ml des so gewonnen Diagnostikums Ratten intravenös appliziert (typische Versuchsergebnisse können der entsprechenden Tabelle entnommen werden).

## Patentansprüche

1. (2,4,5-Trimethylacetanilido)-iminodiacetat.

2. Verfahren zur Herstellung von (2,4,5-Trimethylacetanilido)-iminodiacetat, dadurch gekennzeichnet, daß man Chloressigsäure-(2,4,5-trimethylanilid) in alkalischem Medium mit Iminodiessigsäure umsetzt.

3. Diagnostikum zur Darstellung des hepato-biliären Systems, das ein mit Technetium-99m markiertes Acetanilido-iminodiacetat (IDA) in einem geeigneten Lösungsmittel enthält, dadurch gekennzeichnet, daß das IDA (2,4,5-Trimethylacetanilido)-iminodiacetat ist.

4. Diagnostikum nach Anspruch 3, dadurch gekennzeichnet, daß das Lösungsmittel physiologische Kochsalzlösung ist.

5. Verfahren zurt Herstellung des Leberfunktionsdiagnostikums, gemäß Anspruch 3 und 4 wobei man ein Acetanilido-iminodiacetat (IDA) in wäßriger Lösung mit einer Zinn(II)-Verbindung im Molverhältnis zwischen 10:1 und 200:1 mischt, die Lösung auf einen pH-Wert zwischen 4 und 9, vorzugsweise zwischen 5,0 und 6,5, einstellt und anschließend je nach Verwendungszweck 0,1—100 mCi Technetium-99m-Pertechnetat in einem geeigneten Lösungsmittel, vorzugsweise in physiologischer Kochsalzlösung, zugibt, dadurch gekennzeichnet, daß man als IDA (2,4,5-Trimethylacetanilido)-iminodiacetat (TMIDA) verwendet.

6. Verfahren nach Anspruch 5, dadurch gekennzeichnet, daß die Konzentration von TMIDA zwichen 0,1 und 200 mg/ml, vorzugsweise zwischen 10 und 50 mg/ml, liegt.

7. Verfahren nach Anspruch 5, dadurch gekennzeichnet, daß die wäßrige Lösung von TMIDA und Zinn(II)-Verbindung vor der Zugabe der Pertechnetatlösung in kleinen Portionen (Markierungseinheiten) gefriergetrocknet wird.

8. Verfahren nach Anspruch 7, dadurch gekennzeichnet, daß eine Portion (Markierungseinheit) 1

bis 200 mg, vorzugsweise 10 bis 50 mg, TMIDA sowie 0,01 bis 5 mg, vorzugsweise 0,1 bis 0,5 mg, Zinn(II)-Salz enthält.

9. Verfahren nach den Ansprüchen 5 bis 8, dadurch gekennzeichnet, daß als Zinn(II)-Verbindung $SnCl_2 \cdot 2H_2O$, $SnF_2$, $SnSO_4$, Sn-tartrat, SnO, Sn-oxalat oder Sn-acetat verwendet wird.

**Revendications**

1. (2,4,5-triméthylacétanilido)-iminodiacétate.

2. Procédé de préparation du (2,4,5-triméthylacétanilido)-iminodiacétate, caractérisé en ce qu'on fait réagir du 2,4,5-triméthylanilide de l'acide chloracétique en milieu alcalin avec de l'acide iminodiacétique.

3. Agent de diagnostic pour la représentation du système hépato-biliaire qui contient un acétanilido-iminodiacétate (IDA) marqué par du technétium 99m dans un solvant approprié, caractérisé en ce que l'IDA est le (2,4,5-triméthylacétanilido)-iminodiacétate.

4. Agent de diagnostic selon la revendication 3, caractérisé en ce que le solvant est une solution de chlorure de sodium physiologique.

5. Procédé de préparation de l'agent de diagnostic de la fonction hépatique suivant la revendication 3 ou 4, dans lequel on mélange un acétanilido-iminodiacétate (IDA) en solution aqueuse avec un composé de l'étain (II) dans un rapport molaire compris entre 10:1 et 200:1, on ajuste la solution à un pH compris entre 4 et 9, de préférence entre 5,0 et 6,5, puis on ajoute, suivant l'utilisation envisagée, de 0,1 à 100 mCi de technétium-99m-pertechnétate dans un solvant approprié, de préférence dans une solution de chlorure de sodium physiologique, caractérisé en ce qu'on utilise comme IDA du (2,4,5-triméthylacétanilido)-iminodiacétate (TMIDA).

6. Procédé suivant la revendication 5, caractérisé en ce que la concentration de TMIDA est comprise entre 0,1 et 200 mg/ml, de préférence entre 10 et 50 mg/ml.

7. Procédé suivant la revendication 5, caractérisé en ce que la solution aqueuse de TMIDA et de composé de l'étain (II) est lyophilisée par petites portions (unités de marquage) avant l'addition de la solution de pertechnétate.

8. Procédé suivant la revendication 7, caractérisé en ce qu'une portion (unité de marquage) contient de 1 à 200 mg, de préférence de 10 à 50 mg, de TMIDA ainsi que de 0,01 à 5 mg, de préférence de 0,1 à 0,5 mg, de sel d'étain (II).

9. Procédé suivant les revendications 5 à 8, caractérisé en ce qu'on utilise comme composé de l'étain (II) $SnCl_2 \cdot 2H_2O$, $SnF_2$, $SnSO_4$, du tartrate de Sn, SnO, de l'oxalate de Sn ou de l'acetate de Sn.

**Claims**

1. (2,4,5-trimethylacetanilido)-iminodiacetate.

2. A process for the preparation of (2,4,5-trimethylacetanilido)-iminodiacetate which comprises reacting chloroacetic acid -(2,4,5-trimethylanilide) with iminodiacetic acid in an alkaline medium.

3. A diagnostic agent for visualizing the hepatobiliary system which contains a technetium-99m-labelled acetanilido-iminodiacetate(IDA) in a suitable solvent, wherein IDA is (2,4,5-trimethyl-acetanilido)-iminodiacetate.

4. A diagnostic agent as claimed in Claim 3, wherein the solvent is a physiological saline solution.

5. A process for the preparation of the diagnostic agent for liver function diagnosis as claimed in Claims 3 and 4, which comprises mixing an acetanilido-iminodiacetate (IDA) in aqueous solution with a tin(II) compound in a molar ratio of from 10:1 to 200:1, adjusting the solution to a pH of from 4 to 9, preferably 5.0 to 6.5, and subsequently adding from 0.1 to 100 mCi of Tc-99m pertechnetate according to the purposes in a suitable solvent, preferably in a physiological saline solution, wherein IDA is (2,4,5-trimethylacetanilido)-iminodiacetate (TMIDA).

6. The process as claimed in Claim 5, wherein the concentration of TMIDA is from 0.1 to 200 mg/ml, preferably 10 to 50 mg/ml.

7. The process as claimed in Claim 5, which comprises lyophilizing the aqueous solution of TMIDA and tin(II) compound in small portions (labelling units) before adding the pertechnetate solution.

8. The process as claimed in Claim 7, wherein one portion (labelling unit) contains from 1 to 200 mg, preferably 10 to 50 mg, TMIDA and from 0.01 to 5 mg, preferably 0.1 to 0.5 mg, of tin(II) salt.

9. The process as claimed in Claims 5 to 8, which comprises using as a tin(II) compound $SnCl_2 \cdot 2H_2O$, $SnF_2$, $SnSO_4$, Sn tartrate, SnO, Sn oxalate or Sn acetate.